# EUROPEAN PATENT APPLICATION

(11) **EP 4 548 847 A1**
(43) Date of publication of application: **07.05.2025**
(21) Application number: 23207519.2
(22) Date of filing: 02.11.2023
(51) Int. Cl.: A61B 5/256, A61B 5/369, A61B 5/00, A61N 1/04

(54) **METHODS AND DEVICES FOR IMPROVED EEG SOURCE IMAGING AND IMPROVED ELECTROSTIMULATION OF THE BRAIN**

(71) Applicant: Bottneuro AG, 4056 Basel (CH)
(72) Inventor: OSMANI, Bekim, 4056 BASEL (CH); ALOIS, Hopf, 4056 BASEL (CH); JULIUS, Klaas, 4056 BASEL (CH); MAHYAR, Joodaki, 4056 BASEL (CH)
(74) Representative: Mertzlufft-Paufler, Cornelius

(57) **Abstract**

Several methods and devices are proposed for improved electroencephalography source imaging (ESI) and/or improved and tailored electrostimulation. The approach is based on an electrode helmet (1) that carries a set of electrodes (2) at precisely known and predefined spatial positions, in particular because each electrode (2) is held in place by a respective electrode holder (4) of the helmet (1). As the helmet (1), in particular said electrode holders (4), can be 3D-printed based on 3D design data which are based on patient-specific 3D anatomical data, the helmet (1) offers a platform for patient-specific recording of EEG signals and/or electrostimulation. Accordingly, tedious 3D optical scanning of each electrode (2) position is no longer necessary, such that highly accurate ESI and electrostimulation are possible as soon as the patient is wearing the helmet (1) in the intended well-defined orientation on his head (cf. Figure 2).

## Description

The present disclosure relates to the field of electrostimulation and electroencephalography (EEG). In detail, the disclosure concerns: a method for high-accuracy electroencephalography source imaging (ESI); further a method for preparing an electrode helmet carrying several electrodes, which may be employed for electrostimulation and/or for recording of electrical signals, in particular for recording an EEG-signal; further a method for preparing / enabling a reliable and desired electrostimulation of a brain of a patient; and finally and electrode helmet featuring a controller which can perform an electrical measurement.

In the state of the art, it is common to use EEG-signals (EEG = Electroencephalography) recorded with electrical recording electrodes, to determine the spatial position of the brain region from which the recorded nerve signals originate. This approach is commonly called "EEG source imaging" (ESI). The aim of this method is thus to determine which region inside the brain is currently active and responsible for the momentaneous EEG signal. As the EEG signals vary in time and space, so do the underlying sources within the brain.

It is also known to create patient-specific head models based on MRI data, which show the distribution of electrical conductivity within the skull and brain by means of appropriate segmentation. For example, MRI data from a patient can be used to distinguish between gray and white matter within the brain of a patient, and then literature values for the respective electrical conductivity can be assigned to individual layers of the corresponding head model, which models the head of the patient. With such a patient-specific head model, not only can the size and shape of the patient's brain be considered (which varies from patient to patient), but also patient-specific inhomogeneities of the brain tissue or of the skull. Such a model can also consider patient-specific inhomogeneities of the skull bone and the scalp located on top of the bone. All this is beneficial for more accurately simulating electrical field distributions inside the brain, based on the patient-specific head model.

In the state of the art, however, standard non-personalized head models are typically used for ESI, and these models comprise only average values for the distribution of the individual layers within the patient's head and the respective corresponding electrical conductivities of each layer.

Furthermore, in the state of the art, the approach has been followed so far, in particular for the recording of EEG signals with the aid of electrodes, to always use the same arrangement of electrodes for the patients within a control group. This approach makes the measurements results comparable and also repeatable, which is why it is preferred by clinicians in clinical studies and also in normal treatment/examination of patients.

Such an approach is also followed for example by US 2021 015427 A1, which proposes a helmet with magnetic sensors designed for Magnetoencephalography (MEG) (which is a functional neuroimaging technique for mapping brain activity by recording magnetic fields produced by electrical currents occurring naturally in the brain using sensitive magnetometers): Figure 3 of US 2021 015427 A1 proposes a helmet, in which the outer helmet shell 202 is always retained unchanged and thus the positions of magnetic sensors 203 used for recording MEG-signals remain unchanged, i.e. the magnetic sensors 203 always remain in the same positions on the outer helmet shell. This approach greatly simplifies the computing, because the electrode positions are well-known and do not change. However, this particular helmet can be used on different patient heads for recording of signals, because US 2021 015427 A1 proposes a patient-specific inner helmet shell 201, which is connected to the outer helmet shell 202 via connecting elements 301. The inner helmet shell 201 (but not the outer shell) is patient-specific, i.e., the design of the inner shell 201 is based on the head shape/skull shape of the respective patient and thus fits to one particular head. The spatial relation between the inner shell and the outer shell carrying the magnetic sensors is fixed by the connecting elements, i.e., the position of each sensor is thus known relative to the patient's head.

Thus, in approaches known in the prior art so far, the position of the electrodes/sensors is typically adjusted (if at all) only on the basis of the shape of the patient's skull, i.e., relative to the skull.

There exist also approaches, in which, during an EEG recording session, the electrodes are placed on the patient's head (at almost arbitrary positions) and then the electrodes are elaborately scanned with the aid of a 3D-scanner. By this approach, the position of each recording electrode can be determined reasonably accurately relative to the patient's skull, although the position is at first not known exactly. In principle, the relative position of each electrode relative to the brain could then be extracted in a third step from pre-existing MRI data of the patient's brain.

Moreover, in the state of the art, EEG signals are typically recorded continuously, while the mapping is only performed for a specific point in time. This is because the electrical activity in the brain is dynamic (described by so-called brain waves), such that the field distribution, which is measured with the electrodes, also changes continuously over time.

Starting out from this background, it is an object of the present invention to provide a method and corresponding means for more accurate ESI. Another object is to increase the reliability of an electrostimulation and/or an electro-recording performed using an electrode helmet and also to simplify the overall procedure to allow efficient recording and/or electrostimulation sessions for the patient at home without the need for sophisticated assistance or supervision.

In accordance with the present invention, a method for high-accuracy electroencephalography source imaging (ESI) is provided according to claim 1. In particular the invention proposes a method as introduced at the beginning, which, in addition, is characterized in that a respective electrode position for each electrode of a set of recording electrodes is fixated on an electrode helmet according to an optimization goal (as detailed below) and/or according to a medical objective (e.g. recording an EEG / performing ESI within a particular sub-region of the patient's brain). By this approach, the helmet is/becomes therefore usable for recording the electroencephalography (EEG) signal that can then be used for computing the unknown spatial location and/or extent of at least one electrical source (This particular source is present in the patient's brain and is responsible for the EEG signal). A clinician may choose the location of each electrode according to an assumption or estimate of this source's position within the brain. Furthermore, each individual electrode position can be further optimized according to a specific metric and/or optimization goal.

In this scenario the helmet carries said electrodes, namely at well-known and predefined positions, as determined by said fixation of the electrodes on the helmet. Furthermore, as soon as the patient, for whom the helmet is specifically designed, is wearing the helmet in a correct (as designed) orientation on his skull, the position of each electrode is known relative to the patient's skull and - if corresponding data (such as an MRI-scan) of the patient's brain are available - also relative to the patient's brain. Note that such a helmet may have various forms, in particular in the form of a cap or a bonnet. Important for the method, however, is mainly that the "helmet" is designed in such a way that it can be worn by a particular patient in a well-defined orientation and position such that a respective spatial relation between the skull of the patient and each electrode is well-known as soon as the patient is wearing the helmet on his skull.

If an electrical field distribution on the scalp surface of the patient (i.e. a 2D-field as recorded on a 3D-surface) is electrically recorded by means of the recording electrodes (for this purpose, numerous electrodes must be placed on well-known positions on the patient's head), this field distribution and a patient-specific head model, which defines the conductivities of the individual layers, can be used to perform a so-called "mapping". In such a mapping, the recorded electrical field distribution ("scalp electrical field E(x,y)") is used as input data to calculate the charge distribution present in the volume of the brain, based on the inverse solution of Maxwell's equations. We note that knowing each position of each electrode used in the recording is crucial for accurately deriving the electrical field distribution and for computing the charge distribution inside the brain. In this way, it is thus possible to determine the particular (electrical) source that generates a particular signal in the brain and thus the measured electric field distribution on the scalp, as recorded with the electrodes at a certain point in time. This approach allows a high precision EEG source imaging. A high accuracy often results from using methods of triangulation and from eliminating redundancy, which is possible since numerous recording electrodes are available.

Accordingly, in the method according to the invention, the so predefined electrode positions are considered during/in the (consecutive) computation performed, i.e., when computing said spatial location and/or extent of at least one electrical source (which is the actual step that we may describe as a "source imaging" or "source localization"; source localization / source imaging refers to inferring the distribution of current/charge sources from measured EEG data). Accordingly, this computation is based on the predefined electrode positions and/or uses these positions as input data. Note that the computation is performed after and based on a recorded EEG signal; for example, the computation can be based on an electrical field distribution derived from an EEG signal recorded with the helmet. And the computation may comprise a "mapping" as detailed above.

In more detail, the source-imaging-computation, which may comprise several computational steps, may consider as input data: an EEG signal recorded with the helmet, in particular in the form of an electrical field measured with the set of recording electrodes on the scalp of the patient; the respective position of each electrode relative to the helmet (and thereby also relative to the skull and/or brain of the patient); and an electrical head model of the patient, in particular as detailed below. Based on the recorded EEG signal and each electrode position, an electrical field distribution on the patient's skull can be computed. Based on this field distribution and the head model, a corresponding 3D-charge distribution inside the brain can be computed, in particular in the form of an inverse solution based on Maxwell equations.

And from the charge distribution individual EEG-sources can be identified. As output data, the computation may provide a digital source image or other suitable digital data that allow a meaningful medical diagnosis, further treatment planning, or defining a consecutive electrostimulation of the patient's brain.

Note that according to the invention, this computation can be done without performing any 3D-measurement of the electrode positions, because each position is inherently known from the design of the helmet. This shows the great advantage of this method as compared to previous approaches, in which tedious 3D-measurements are required for each ESI-session.

The new approach of the invention therefore provides that, for example on the basis of MRI data, first of all a suitable respective electrode position is determined. In other words, for each electrode that is to be used later during the EEG recording its spatial position on the electrode helmet is predetermined, in particular according to the needs of a particular patient for whom the helmet is designed. For example, a clinician can decide based on MRI or other data which region of the patient's brain merits closer inspection by recording of an EEG signal or which part of the brain shall be treated by an electrostimulation. Accordingly, the clinician can choose the positioning of the electrodes on the helmet according to a medical objective. This positioning can be coarse, and in a second step, a finer positioning of each electrode can be calculated/optimized, based on an optimization goal.

Subsequently, an electrode helmet/electrode cap may be 3D-printed on the basis of such determined electrode positions, and it is ensured (by the specific shape of an inner contour of said helmet) that the patient can only wear the helmet in a certain position and orientation on his skull. By this concept, the same electrode position relative to the patient's brain is repeatedly and reproducibly achieved as soon as the patient puts on the helmet (in the correct orientation). In other words, all electrode positions can be fixed, once the helmet is fabricated, but on spatial positions on the shell of the helmet, which have been chosen to specific needs of the patient.

Hence, the invention proposes a patient-specific helmet with patient-specific arrangement/ spatial placement of the electrodes. This novel approach has the considerable advantage in comparison to the state-of-the-art that a 3D scan does not have to be performed again for each new EEG recording session, in order to determine the electrode positions relative to the skull/brain in each new session. Instead, each electrode position relative to the patient's skull and also relative to the patient's brain is well known as soon as the patient puts on the helmet (i.e., as soon as the patient is wearing the helmet in the intended orientation on his skull).

We also note that the fixation/setting of the electrode positions on the helmet (thereby fixing the position of each electrode relative to the helmet and - as soon as the helmet is worn by the patient - also relative to the patient's skull and brain) is completed prior to performing EEG recording using the set of recording electrodes. For example, the predefined/fixed positions of the electrodes on the helmet can be generated/computed while 3D modeling the head of the patient, for example based on MRI images. The determination of the positions can be based on various boundary conditions, in particular optimizing a specific metric (as detailed below) and/or meeting a specific medical objective (e.g., focusing the recording and/or later stimulation on a particular region of interest (ROI) within the brain).

We further note that the method according to claim 1 does not necessarily comprise the step of actually recording the EEG signal (a step which is performed with the electrodes being in contact with the skull on the head of the patient wearing the helmet). However, this EEG-recording may be part of a medical procedure. We note that the method proposes a particular arrangement of the electrodes on an electrode helmet and a particular source computation (based on an already recorded EEG signal) which considers this particular arrangement.

A medical procedure for improved ESI, which is based on the method, if applied fully, can, however, comprise the following steps:
- a first step, in which an electroencephalography (EEG) signal is recorded using a/the set of recording electrodes and
- a second step, in which a spatial location and/or extent of at least one electrical source responsible for the EEG-signal is computed from the EEG-signal. Preferably the spatial location and/or extent of the at least one electrical source may be outputted as a digital source image.

In summary, the full method/medical procedure may be described as a:
**Method for high-accuracy EEG source imaging (ESI)**, wherein
- in a first step, an EEG signal is recorded using a set of recording electrodes carried by an electrode helmet and
- in a second step, a spatial location and/or extent of at least one electrical source responsible for the EEG signal is computed from the EEG signal, preferably and outputted as a digital source image, **characterized in that,**
- according to an optimization goal and/or according to a medical objective, a respective electrode position for each electrode of the set is fixated on an electrode helmet, the helmet carrying said electrodes, and
- the so predefined electrode positions are considered during the computation of the spatial location and/or extent of the at least one electrical source (= "source imaging") performed, in particular without performing any 3D-measurement of the electrode positions.

The digital image data (source images) which may be computed with the method may form the basis for a consecutive planning of a treatment, in particular an electrical neurostimulation, or for a diagnosis performed by a doctor. The method itself however can only be directed towards collecting / producing the relevant source image data allowing such consecutive steps.

A method according to the invention may thus only comprise "an examination phase, involving the collection of data" but not "(ii) a comparison of these data with standard values, (iii) any finding of any significant deviation, i.e. a symptom, during the comparison, or (iv) an attribution of such an deviation to a particular clinical picture, i.e. a deductive medical decision phase (diagnosis for curative purposes stricto sensu)."

Accurately knowing the position of each of the recording electrodes relative to the brain is a basic requirement for an accurate EEG source imaging / an accurate mapping (as detailed above) with sufficient precision (in the meaning of repeated accuracy in consecutive treatment / recording sessions). Based on MRI image data, the respective anatomical position and shape of the patient's brain within his skull can be accurately determined, and this information can be considered when determining the individual position of each of the recording electrodes on the helmet shell when manufacturing the helmet, in particular electrode holders fixing the respective electrode to the helmet's shell.

The approach according to the invention also offers advantages for the recording of nerve signals (brain waves) in the brain. For as long as the relative position between the electrodes and the brain is known, which is possible due to the approach according to the invention, it can be concluded, based on the recorded electrical signals, much more precisely in which anatomical area of the brain the recorded signals originated. In other words, the source of an EEG-signal within the brain can be located with higher spatial resolution allowing more detailed analysis and diagnosis.

With respect to EEG source imaging, the approach according to the invention offers advantages, because due to the knowledge of the relative position of each EEG recording electrode relative to the brain, the respective anatomical region within the brain from which the EEG signal originates can be determined much more precisely. The approach according to the invention can therefore significantly improve the efficiency of EEG source imaging. In addition, it also seems conceivable that a similarly good localization of the relevant brain region can be achieved with a smaller number of electrodes, when following the approach according to the invention.

There are therapy approaches in which phases of electrostimulation alternate several times with phases of recording electrical signals from the brain. In other words, patients may be sequentially stimulated, for example with an electrode helmet as described herein, and afterwards nerve signals are recorded, in particular with the same helmet. In such applications, the approach according to the invention offers advantages, because due to the knowledge of the relative position of the electrodes in relation to the patient's brain, it is possible to check much more precisely whether the area of the brain, which is currently being electrically stimulated, shows the desired higher/lower activity. This enables a patient-specific verification of the effectiveness of a targeted electrical neurostimulation applied by means of an electrode helmet. Another advantage is that a quick change between EEG recording, ESI and electrostimulation is possible, without any need for taking the helmet of the patient's head. With the approach according to the invention, the relative position of each electrode in relation to the brain can be kept the same or at least highly similar for a control group of different patients, which is beneficial for clinical studies. However, if one were to compare the different helmets between the patients, it would be noticed that the absolute positions of the electrodes on the electrodes on the helmet differ, because each patient has a specific skull shape and a specific shape and location of their brain within the skull.

With respect to neurostimulation, the approach according to the invention is also of advantage: For the likely case that two patients have almost identical skull shape, but the position and/or shape of the respective brain within the skull differs, the approach according to the invention leads to a precise stimulation of the desired brain area in both patients (even when using identical electrode arrangements, because the electrical stimulation can be precisely steered to the region of interest within the brain by varying the electrical signals applied during stimulation), whereas with previously known approaches, a sub-optimal stimulation would be expected in such a case.

Effects like atrophies inside the brain, which may arise in the brains of elderly patients, can also be considered when determining the placement of each electrode on the helmet.

The method as laid out before may be elaborated in various ways, as will now be described in detail:
For example, the electrode positions can be determined based on existing patient-specific 3D anatomical data measured from a patient's head, in particular anatomical data describing the spatial distribution of the patient's brain, skull and scalp. Preferably, such 3D anatomical data are measured using a medical imaging technique, most preferably using magnetic resonance imaging (MRI). This approach according to the invention thus results in a new imaging technique w.r.t. the location of an EEG source, which is comparable to (but should not be mistaken as) a functionalized MRI: The knowledge of the exact coordinates of each individual electrode (relative to the brain) used for recording the "EG i' a basic prerequisite for performing the ESI with sufficient accuracy afterwards.

The mentioned computation can preferably use a patient-specific head model which is derived from, in particular the above mentioned, 3D anatomical data measured from a head of a patient. Of course, this approach makes most sense, when the brain of said patient is imaged with the method to find at least one EEG source.

For solving the above mentioned problem, we also propose a corresponding method for preparing an electrode helmet, namely to render the helmet ready for (i) patient-specific electrical stimulation of a particular region of interest (ROI) inside the brain of a patient and/or for (ii) recording of nerve signals emanating from inside the brain of a patient. Each time (during recording and/or stimulation), a respective set of electrodes, which is carried by said electrode helmet, is used. In fact, the helmet may carry two different sets of electrodes, one designed for recording, the other designed for stimulation. However, it is also possible to use at least some or all of the electrodes first as a recording electrodes and later as a stimulation electrodes. For solving the problem, the method further comprises the following steps:
- step A): deriving 3D design data for the helmet, including respective electrode positions for each electrode of the set, from existing patient-specific 3D anatomical data measured from the patient's head. Of course, such a measurement can be performed to provide such data. We also note that the electrode positions can be based on an optimization goal and/or according to a medical objective, as already mentioned.
- step B): arranging and/or mounting a number of m electrodes on the helmet in a patient-specific and/or customized arrangement that is defined by or derived from the 3D design data obtained in step A). In other words, the 3D design data obtained in step A) can define each electrode position on the helmet.

We note that the fact that the electrodes have been placed not arbitrarily but by purpose will be hard to detect by inspecting the helmet alone. However, typically, there will exist a digital file or other data which define the position of each electrode on the helmet, and such "placement data" (in particular as part of said 3D design data) will depend on the patient-specific 3D anatomical data measured from the patient's head. Accordingly, the method of preparing said helmet can include the generation of "placement data", which describe each position of each electrode to be employed on the helmet.

The purpose of an electrostimulation provided by such a helmet can be to provide a tailored electrical stimulus to a specific region of interest (ROI) located deeply inside the patient's brain. By contrast, with such a prepared helmet an EEG recording can be performed on the whole head of the patient without knowing at first, where the source to be imaged is exactly located inside the brain. In other words, such EEG recording can have the purpose to identify the spatial location of a particular source region within the brain for a first-time, based on the knowledge of the relative distance and location of the electrodes on the helmet w.r.t. each other and w.r.t. anatomical structures/regions inside the patient's brain. Afterwards, the so-located source inside the brain may be purposely targeted and electrostimulated using the helmet. One major advantage of the approach is thus that a highly accurate and patient-specific electrical stimulation of a certain ROI inside the patient's brain and/or a highly accurate EEG source imaging (by recording nerve signals using the predefined arrangement of the electrodes) can be performed after the helmet has been prepared. A patient can put the helmet on his head and even perform the recording and/or stimulation autonomously, without any assistance from clinical stuff, in particular at home. And most importantly, no time-consuming, laborious and complicated 3D scan requiring a sophisticated 3D-scanning setup needs to be performed prior to the stimulation and/or recording session, as was previously often the case.

With this new approach, it is thus possible to carry out repeated EEG recording sessions in which the patient only has to put on the helmet, and wherein subsequently - on the basis of the precisely known and always constant electrode positions - a mapping can be carried out on the basis of the electrically recorded field distribution on the scalp of the patient to repeatedly implement a respective precise electrical source imaging (ESI).

To safeguard that the electrode positions remain the same relative to the patient's brain, it is of advantage, if the helmet is fabricated with a patient-specific geometry such that the helmet fits the skull of one particular patient accurately. Based on such patient-specific design of the helmet, in particular of the helmet's inner side/inner shell facing the scalp of the patient, the position of each electrode will be well-defined with respect to a sagittal and a frontal plane running through the head of the patient wearing the helmet, and thereby relative to the patient's brain. We note, that the brain can slightly move inside the skull of the patient due to the presence of the cerebrospinal fluid (CSF), but will normally resume an equilibrium position which may be known from the measured 3D anatomical data. Hence each electrode position will be well-known w.r.t. this equilibrium position.

The approach of the invention can also form the basis of a specific method of therapy and/or diagnosis as laid out in the following: First, in step A), a number of m electrode positions for each electrode of the set are predefined (and thereby fixed) based on the 3D design data derived from existing patient-specific 3D anatomical data measured from the patient's brain. Next, the electrode helmet is fabricated, in particular using 3D-printing, and/or assembled and a number of m electrodes are arranged, in particular mounted, on the helmet (for this purpose, special electrode holders may be employed, in particular as part of the helmet's shell) in a desired patient-specific arrangement that is defined by the 3D design data derived in step A). This arrangement can be based on a specific ROI to be stimulated or based on a certain ROI from which EEG source signals are to be recorded.

And finally, in a step C), the so prepared electrode helmet can be worn by the patient and an electrostimulation (with the purpose of achieving a therapeutic effect) of the patient's brain (electrical neurostimulation using DC or AC currents) can be performed using the set of electrodes as electrical stimulation electrodes and/or an EEG recording and source imaging (ESI) can be performed using the set or a second set of electrodes (each set being defined by the 3D design data) as electrical recording electrodes. As such stimulation and/or recording (as performed in step C) requires no time-consuming 3D-scanning of positions of the helmet and/or of positions of the electrodes, the patient can perform stimulation and/or recording sessions using the helmet autonomously at home.

For the EEG recording performed in step C), a set of 32 electrodes can already result in a reasonable spatial resolution of the EEG; however, conceivable are up to 256 electrodes or even more that may be distributed over the patient's scalp / may be carried by said helmet. In addition, additional recording electrodes may be placed on the face of the patient, as is known in the art.

During the "mapping" as part of the ESI, a so-called "freeze" of the underlying models can be performed, by which the spatial distribution of the electrical sources within the brain can be calculated at a certain point-in-time. Such mapping / ESI may be performed repeatedly in short time intervals, thereby enabling recording of the transient behavior of each source (i.e., resolving how the EEG sources change their activity and/or location over time).

The ESI may be performed using a head model, in particular including (e.g., standardized) spatial distributions of electrical conductivities, as typically found within a human head, for example including conductivities for scalp, skull, brain, blood, muscles, etc. (which are all available from literature or from experiments).

Most preferably, however, the invention proposes to employ a personalized (i.e., patient-specific) head model, when deriving the 3D design data in step A) and/or when performing ESI in step C). Thereby the invention contributes to making high-precision ESI more broadly available for patients, in particular in applications in which the ESI is performed by the patient herself/himself. According to the invention, such a patient-specific head model can be based on/derived from said 3D anatomical data. In other words, such a patient-specific head model can comprise multiple spatial distributions of different layers, each with assigned (e.g., standard / literature) respective electrical conductivity, with each layer corresponding to a respective tissue and/or bone layer as visible in the 3D anatomical data.

The mentioned 3D anatomical data, which may form the basis for fixing the positions at which the electrodes are placed on the helmet, can be or can have been measured using a medical imaging technique. Most preferably, the 3D anatomical data may be obtained, at least in part, using magnetic resonance imaging (MRI), and even more preferably using diffusion-weighted magnetic resonance imaging (DWI).

One implementation suggests that the anatomical data comprise image data measured with at least two different medical imaging techniques. For example, a first set of data may be measured with diffusion-weighted magnetic resonance imaging (DW-MRI) and a second set of data may be measured with structural magnetic resonance imaging. Diffusion-weighted magnetic resonance imaging (DW-MRI) uses specific MRI sequences as well as software to generate images from the resulting data that are based on the diffusion of water molecules to generate contrast in the MR images. DW-MRI allows the mapping of the diffusion process of molecules, mainly water, in biological tissues, in vivo and non-invasively. Structural magnetic resonance imaging (MRI), on the other hand, is a non-invasive technique for examining the anatomy and pathology of the brain, as opposed to using functional magnetic resonance imaging [fMRI] to examine brain activity.

The 3D anatomical data may also comprise further data, for example derived from a computed tomography (CT) scan.

The approach of using multiple data sets is particularly of advantage, because based on such extensive anatomical data a highly accurate patient-specific head model of the patient's brain can be derived, in which the spatial location and spatial distribution of soft matter layers (such as white and gray matter within the brain) and of hard matter layers (such as bone) within the patient's head are comprised. Such a sophisticated head model can be beneficial for more accurately determining said electrode positions in step A) with the aim of a precise electrostimulation of a certain ROI; and it can be beneficial for more accurately performing an ESI in step C), using the electrode positions defined in step A).

The respective electrode positions for each electrode (no matter whether it is later used for recording and/or for electrostimulation) of the set may be calculated based on a patient-specific head model which is derived from said 3D anatomical data. This may be performed in particular in step A, when preparing the helmet. It is further preferable, if said patient-specific head model comprises multiple spatial distributions of different layers, wherein a respective electrical conductivity (e.g., standard / literature values or empirically found values) is assigned to each layer. Each layer may correspond to a respective tissue layer or bone layer as visible in the 3D anatomical data. The mentioned spatial distributions of the different layers within the brain are often termed "segments". Accordingly, the head model may be a segmented head model, which is generated by segmentation of the 3D anatomical data. There are methods available for computer-implementing such segmentation.

Today, average models are widely used for modeling the scalp conductivity, which can have a major impact on the recorded signals and/or on the effectiveness of an electrostimulation. In principle, the distribution of the individual biological tissue layers (bone, spongiosa/spongy bone, scalp) can be determined relatively accurately using MRI data; however, the conductivity of these layers varies from patient to patient and may even change for a particular patient over time, depending on the situation (e.g., when the patient starts to sweat or depending on the blood flow to the respective tissue). Accordingly, current models for the scalp conductivity often fail to reflect the true momentary conductivity of the scalp of the patient, resulting in a possible ineffectiveness of a desired electrostimulation and or inaccurate ESI, because the underlying model is not accurate enough.

According to a separate aspect of the invention, the following method is proposed for determining the momentary conductivity of the scalp accurately, which may result in a "fined-tuned" / more accurate patient-specific head model:
In a first step i), a known brain activity is stimulated in the brain of a patient. This may be done a) by instructing the patient to perform a certain motoric/body movement (for example, lifting her/his arm in a defined way). Such a movement will then result in a specific nerve signal within a specific region of the brain (the cortex). This ROI may then be identified within said existing 3D anatomical data measured from the patient's brain previously. For example, within existing MRI data of the patient's brain, the ROI may be identified and located very precisely. Alternatively, such defined brain activity may be stimulated b) by an external nerve stimulator placed outside the brain, in particular by means of stimulation electrodes carried by an electrode helmet worn by the patient on his head. In other words, the brain activity could be stimulated by an electrostimulation, in particular performed using a set of stimulation electrodes carried by said electrode helmet.

In a second step ii), an electrical field distribution arising from the stimulated brain activity is measured by a set of recording electrodes, in particular in the form of an EEG (EEG recording). This recording may be performed using an electrode helmet as disclosed herein.

Since the ROI responsible for the electrical activity within the brain is already known (because the activity has been purposedly stimulated), the source of this activity, more precisely its location inside the brain, can be localized independent of the recorded EEG. This may be done most accurately using existing patient-specific 3D anatomical data measured from the patient's brain, in which the anatomical region of interest responsible for the stimulated nerve-signal can be located within space. Hence, based on anatomical knowledge and existing data, a suspected (spatial) source position and extension of the source (in accordance with the stimulation performed) can be derived, and in a third step iii) this suspected source position and/or extension can then be compared with a calculated source position and/or extension, as computed on the basis of the recorded brain activity, more precisely on the basis of the recorded electrical field distribution arising from the stimulated brain activity, and based on a patient-specific head-model (as already detailed above). Most accurately, this calculated source position and/or extension may be determined by ESI, considering a current version of the patient-specific head-model.

In this way, a correction loop can be set up in a fourth step iv) in which the patient-specific (electrical) head model (which may model the electrical conductivities of the scalp in particular) is adjusted iteratively until the calculated source corresponds as closely as possible to the suspected source. In other words, several configuration parameters of the head model may be fine-tuned based on a respective comparison between the calculated source and the suspected source; and these steps can be iteratively repeated, to narrow the gap between the calculated source and the suspected source. Through this approach, a head model can be obtained that most accurately matches the momentary distribution of conductivities within the head of the patient. Afterwards, more accurate ESI or electrostimulation may be performed, using the so optimized patient-specific head model.

In summary, this aspect thus proposes a
**Method for obtaining a patient-specific head model,** comprising the following steps:
i) a known brain activity is stimulated in the brain of a patient, in particular by instructing the patient to perform a certain motoric/body movement or by an external, preferably electrical, nerve stimulator;
ii) an electrical field distribution arising from the stimulated brain activity is measured by a set of recording electrodes, in particular in the form of an EEG;
iii) a suspected source position and/or extension is compared with a calculated source position and/or extension, as computed on the basis of of the recorded electrical field distribution arising from the stimulated brain activity, and based on a patient-specific head-model;
iv) the patient-specific head model used in step iii) is adjusted iteratively until the calculated source corresponds as closely as possible to the suspected source, in particular by fine-tuning configuration parameters of the head model.

For example, the calculated source may correspond approximately to the position of the cortex. The simulation/calculation can then be adjusted until the calculated source corresponds exactly to the spatial area identified in existing MRI data from the patient's brain as the patient's cortex. The adjustment may change parameters of the head model as well as parameters of the calculation routine applied (e.g., by changing assumptions on certain boundary conditions). The adjustment/adaptation of a conductivity model used in said calculation / of said head model can preferably be carried out with the aid of a computer simulation, in which the respective model is optimized until the calculated source corresponds as closely as possible to the suspected source.

The adjustment of the electrical head model can be performed dynamically, for example at the beginning of each ESI session or even during an EEG recording, in any case at least at two points in time. Thereby, momentary changes in the conductivity, for example of the scalp of the patient, may be accurately modeled and considered.

Most preferably, the stimulation of the brain activity may be performed with a first set of stimulation electrodes carried by said helmet and the electrical field distribution resulting from the stimulated brain activity may be recorded with a second set of recording electrodes also carried by said helmet. Such an approach can be helpful for improving the accuracy in the later determination of another source within the brain producing a different electrical field distribution. It is therefore presupposed that from the predefined respective positions of the electrodes and the relative arrangement of the two sets of electrodes to each other on the electrode helmet, it is already sufficiently determined, which region of the brain is electrostimulated and afterwards electrically recorded/imaged by ESI. This region of interest can then be linked to existing anatomical 3D-data (such as MRI data) to achieve the desired auto-/fine-tuning of the electrical head model.

As mentioned, the respective electrode positions for each electrode of the set can be calculated based on an optimization goal. This may be done in particular during step B, when preparing the helmet. Such a goal may be:
- maximizing the effectiveness of an electrical stimulation of at least one pre-defined region of interest inside the patient's brain and/or
- maximizing the resolution when recording EEG signals from a particular source/region of interest within the patient's brain.

The ROI may have been determined/specified based on pre-existing image data measured with various medical imaging techniques such as MRI, CT-scans or DWI. The determination / specification of the ROI may be performed by a doctor or using some elaborated artificial intelligence (AI) which analyzes pre-existing images of the patient's brain and identifies possible ROI.

When preparing the helmet, in particular during step B), at least parts of said helmet may be 3D-printer for fixing/ predefining each of said electrode positions with respect to the brain of the patient for whom the helmet is custom-made. For example, the 3D-printing can produce an inner contour, in particular an inner shell, of the helmet that fits the skull of one particular patient. At the same time, the 3D-printing can produce electrode holders, which define the position of each electrode that is later introduced in each holder.

In other words, step B) may comprise defining (in particular by 3D-printing or by adapting parts of said helmet) an inner contour surface of said helmet based on a specific shape and size of the skull of the patient. Such customization can be such that the helmet can only be worn by the patient on her/his head in an unchangeable orientation and/or position relative to the skull of the patient. Hence, the helmet will show a patient-specific design in this case.

As already noted, the electrodes can be mounted in electrode holders. These holders can be provided separately or as part of a shell of said helmet (in which case they will be integrally formed, in particular 3D-printed, with the shell).

The shape and/or position and/or orientation of each of the electrode holders can be defined by said 3D design data. For example, a shell of the helmet may feature a number of N electrode holders each designed for holding a respective electrode in place. The shell may carry, however, a number of m ≤ N electrodes. Each holder and/or each electrode may be arranged on the shell based on the 3D design data. Such a helmet can thus enable patient-specific electrical stimulation and/or recording of a particular ROI identified within the anatomical 3D data of the patient's brain.

For solving the problem mentioned at the beginning, we further propose a method for preparing a reliable and desired electrostimulation of a brain of a patient. Note that this method must not necessarily comprise the actual step of electrostimulation, but can merely provide the starting point for such therapy: In other words, prior to or simultaneous to performing electrostimulation with a set of stimulation electrodes (which are preferably carried by an electrode helmet, in particular as described herein), an electrical surface conductivity measurement (preferably in the form of an electrical impedance measurement) is carried out using at least one source electrode and at least one electrical sensing electrode (which are preferably carried by said electrode helmet; note that the source and/or sensing electrodes can be employed as stimulation electrodes afterwards). During this measurement, the at least one source electrode injects a known electrical measurement current into the scalp of the patient (i.e., an the measurement current is delivered to the source electrode, thereby injecting the current) and a voltage or current is recorded/ measured by the at least one electrical sensing electrode. This may be done in particular, using a so-called four-probe-measurement-scheme, as described herein.

Of course, it is highly preferably if the so measured electrical surface conductivity is then considered in a consecutive electrostimulation performed with the electrode helmet afterwards. This consideration may be done by adapting / correcting at least one stimulation current delivered by the electrode helmet based on the electrical surface conductivity measurement.

It is also preferable, if the helmet carries at least two electrical sensing electrodes, because this allows computation of a 2D-map of the surface conductivity based on voltages or currents recorded by the at least two electrical sensing electrodes. Accordingly, an electrostimulation performed afterwards (in particular with the same electrode helmet) can consider this 2D-conductivity map by adapting / correcting at least one stimulation current based on the measured 2D-conductivity map.

The at least one source electrode used for injecting an electrical current into the scalp of the patient may be at least one separate electrode or at least one of said stimulation electrodes. Likewise, the at least one sensing electrode may be at least one separate electrode or at least one of said stimulation electrodes.

In practical applications, approximately 90 % of the electrical current delivered to the scalp by a stimulation electrode does not reach the brain at all, but flows through the scalp to an adjacent electrode, for example. Using the at least one source electrode for injecting a specific electric current into the scalp, and at least one electrical sensing electrode (preferably a plurality of such electrical sensing electrodes, arranged in a precisely predetermined geometrical pattern relative to one another on the electrode helmet and thus relative to the skull and brain) for carrying out an electrical impedance measurement, a momentary electrical conductivity on the surface of the scalp can be determined. This is because this surface conductivity of the scalp is precisely responsible for how much current actually reaches the brain. The higher this conductivity, the less electric current will be conducted into the brain and thus be effective for electric stimulation of deep lying brain tissue.

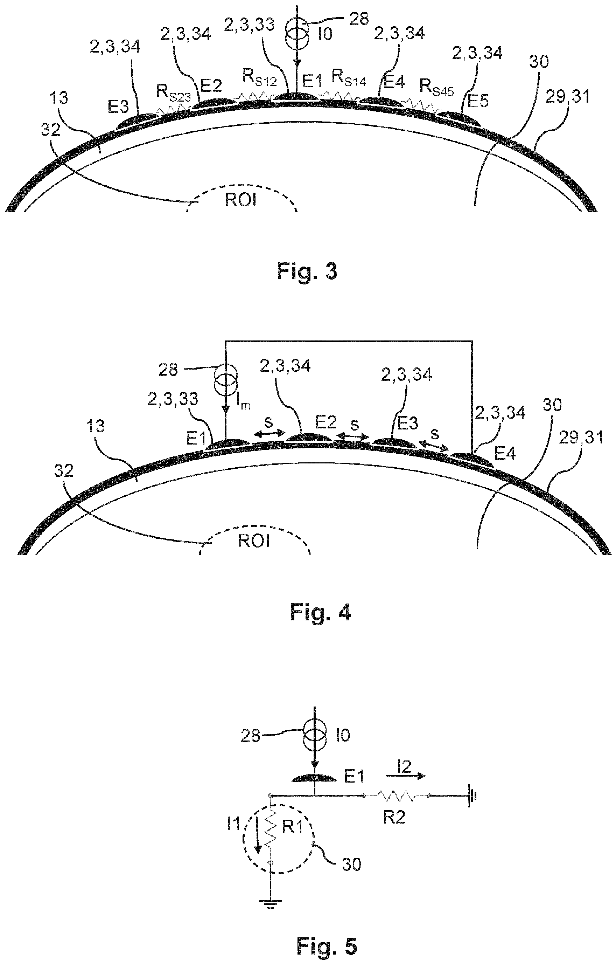

According to one specific embodiment of the method, a plurality of sensing electrodes, which are employed during said measurement, are arranged in a precisely predetermined geometrical arrangement relative to one another on the electrode helmet and thus relative to the skull and brain of the patient. In particular four electrodes of the helmet may be arranged in a four-probe-measurement-arrangement, as described below with respect to Figure 4. Accordingly, a spatial distribution of a surface conductivity (in particular as a 2D conductivity map as mentioned before) or a sheet resistance of the scalp of the patient can then be calculated from the measurement by considering said geometrical arrangement.

The technical advantage of this approach is that the consecutive electrostimulation can consider the results of the measurement, in particular said distribution of a surface conductivity, and can thus adjust the electrical currents used during the electrostimulation. In other words, the method can comprise a consecutive step of electrostimulation, in which at least one electrical stimulation current (as delivered by one of said stimulation electrodes) is adjusted based on the result of said surface conductivity measurement, in particular said impedance measurement.

Finally, for solving the problem we also propose an electrode helmet that offers a patient-specific design (as already detailed above). This helmet carries a set of stimulation electrodes designed and configured for producing an electrostimulation inside the brain of a patient (when this patient is wearing said helmet on his head) and the helmet may be prepared for patient-specific electrical stimulation with a method as described or claimed herein. Moreover, the helmet can feature at least one source electrode and at least one electrical sensing electrode and an electronic controller.

Different from the state of the art, the controller may be configured to perform an electrical surface conductivity measurement, preferably an electrical impedance measurement, using said at least one source electrode and the at least one electrical sensing electrode. In fact, the controller can be configured to perform a four-probe-measurement using four electrodes, as described herein. In other words, the helmet, thanks to these features, is then capable of autonomously measuring and/or monitoring a momentary surface conductivity and/or sheet resistance of the scalp of a patient who is wearing the helmet on his (bare) head. The technical advantage is that such a surface conductivity or sheet resistance measurement performed by the helmet can then be considered when performing electrostimulation of the brain of the patient using the very same helmet, i.e., the controller can automatically adjust at least one stimulation current based on the measurement result.

Accordingly, the controller may be configured to implement a method according to one of the claims 12 to 14, i.e., the helmet, more precisely its controller, can autonomously prepare a reliable and desired electrostimulation to be performed on the brain of the patient wearing the helmet. For this purpose, the controller may consider measurement data obtained in said electrical surface conductivity measurement / electrical impedance measurement / said four-probe-measurement and autonomously adapt / correct at least one stimulation current, which is delivered by stimulation electrodes carried by the helmet. For example, the controller may control individual current sources that provide a respective electrical stimulation current to the respective stimulation electrode of the helmet. In more detail, the controller may be configured in such a way that the controller repeatedly and/or dynamically adjusts at least one electrical stimulation current delivered by one of said stimulation electrodes. Such adjustment may be performed by the controller shortly before or even during an electrostimulation performed with the helmet (in particular each time by considering/based on a momentary result of said measurement).

Preferred examples of the present invention shall now be described in more detail, although the present invention is not limited to these examples: for those skilled in the art, it is obvious that further examples of the present invention may be obtained by combining features of one or more of the patent claims with each other and/or with one or more features of an example as described or illustrated herein.

With reference to the accompanying drawings:
- Fig. 1: is a schematic illustration of an approach for electroencephalography source imaging (ESI) as known from the prior art,
- Fig. 2: is a schematic illustration of the approach according to the invention for patient-specific EEG recording and/or stimulation using an electrode helmet,
- Fig. 3: illustrates a cross-sectional view through the skull of a patient with an arrangement of electrodes 2 attached to the scalp that can be used with a method according to the invention,
- Fig. 4: illustrates a similar cross-sectional view as the one of Figure 3 with electrodes used in a four-point-probe-measurement according to the invention,
- Fig. 5: is a schematic highly simplified equivalent circuit diagram illustrating the effect of the surface conductivity of the scalp on the part of the injected stimulation current reaching deeper layers of the brain,
- Fig. 6: illustrates an electrode helmet that may be used for implementing the method according to the invention, and
- Fig. 7: shows a complex electrostimulation device comprising the helmet of Figure 6.

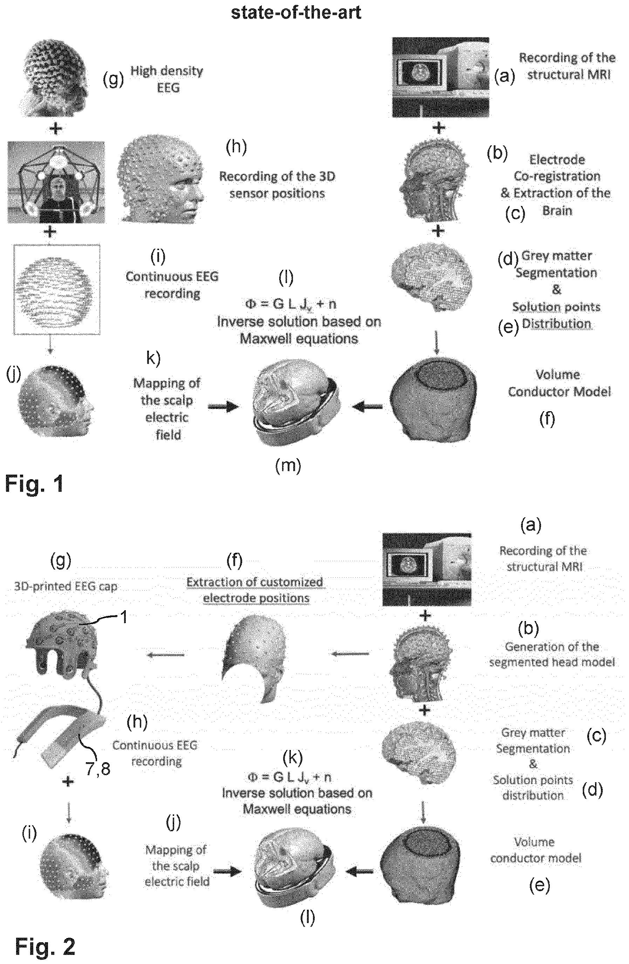

Figure 1 illustrates schematically steps that are necessary for accurately determining the position of a source of an electroencephalography (EEG) signal inside the brain of a patient using a high-density EEG recording. The EEG signals are recorded by multiple recording electrodes which are manually glued onto the head of the patient in high spatial density, which is a rather tedious and unpleasant procedure.

To complicate things even more, each electrode position will vary such that it needs to be first determined, even if the spacing between the electrodes is equidistant. The determination of each electrode position relative to the patient's skull is achieved (in the state-of-the-art) by optically scanning the head of the patient, thereby locating each electrode on the head of the patient in space.

Accordingly, in Figure 1, the single steps are / the single sub-images indicate:
(a) recording of a structural magnetic resonance image (MRI) of the patient's skull and brain;
(b) co-registration of each electrode; this step means that the optically recorded electrode positions may be incorporated into the head model derived from the MRI data;
(c) extraction of brain layers from the MRI data;
(d) gray matter segmentation for producing a segmented head model;
(e) solution points distribution
(f) Creation of a volume conductor model of the patient's head (= head model);
(g) a set of electrodes for high density EEG recording is applied to a patient's head, in particular directly;
(h) measuring of the electrodes 3D-positions using an optical 3D-scanner;
(i) continuous EEG recording using the set of electrodes;
(j) calculation of an electrical field distribution on the patient's skull based on the recorded EEG signal;
(k) mapping of the electrical field, as recorded on the scalp of the patient's head ("scalp electrical field") using the set of electrodes, into a corresponding charge distribution inside the brain;
(l) calculating the charge distribution present in the brain and responsible for the electrical field, based on the inverse solution of Maxwell's equations;

We note that steps (k) and (1) can be performed within a single computational step and that the charge distribution and/or an electrical field distribution inside the patient's brain can be computed point by point (for example for individual Voxels of 1 mm³ in size). We also note that the spatial resolution of the calculation performed in steps (k) and (1) may also be varied (i.e. an uneven/irregular spatial resolution may be used).

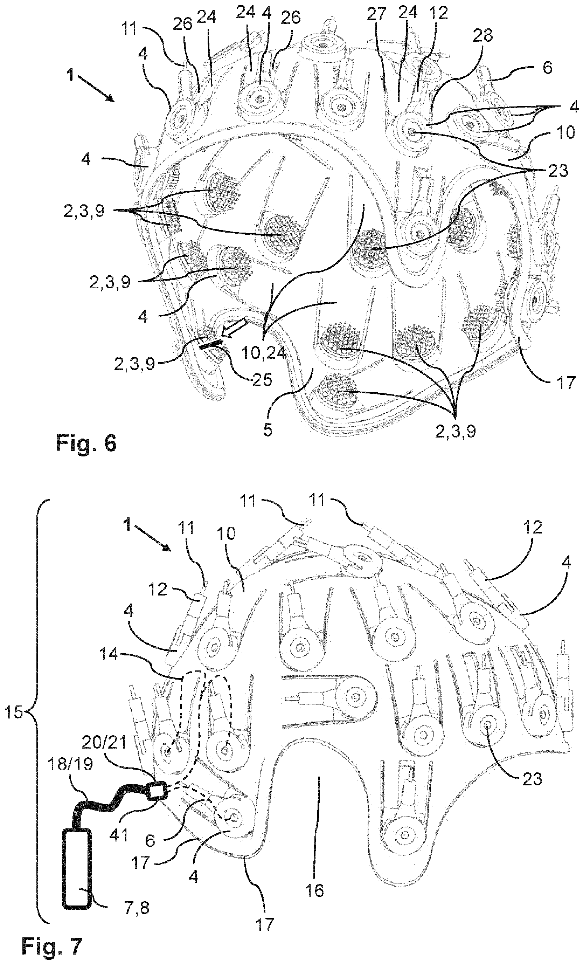

Figure 2 presents an approach according to the invention for a highly accurate ESI using a 3D-printed electrode helmet 1 carrying a set of EEG-recording electrodes 2. Such a helmet 1 is illustrated in Figure 6. The electrodes 2, which are designed as brush electrodes 9, are accurately held in place relatively to the skull of the patient wearing the helmet 1 by respective electrode holders 4. The electrode holders 4 are formed as integral parts of the 3D-printed shell 5,10 of the helmet 1.

The electrodes 2 can also be used as stimulation electrodes. In other words, the helmet 1 may form the basis of a complex electrostimulation device 15, as shown in Figure 7, comprising also an electronic unit 8, which may provide electrostimulation currents and/or control signals for addressing current sources 28 used in the electrostimulation and which can also record EEG signals using said electrodes 2. A controller 7 implemented by the electronic unit 8 can also communicate wirelessly with external devices for receiving commands and/or for transmitting measurement data.

The helmet 1, more precisely the electrode holders 4, also each provide a customized contact force 25 that presses each electrode 2 into the scalp 31 of the patient's head, thereby enabling a sufficiently low electrical contact resistance and a situation as schematically illustrated in Figure 3. For the same purpose, the holders 4 are equipped with feed channels 23 through which a conductive gel can be applied to each brush electrode 9 for further lowering the contact resistance. Each electrode 2 is electrically contacted either by an integrated wiring 14 or by cables attached to the respective electrode connector 12 shown in Figure 6 and 7.

In Figure 2, the single steps performed to obtain a highly accurate ESI are / the single sub-images indicate:
(a) recording of a structural magnetic resonance image (MRI) of the patient's skull and brain;
(b) generation of a segmented head model;
(c) extraction/determination of customized electrode positions;
(d) 3D-printing of electrode cap/helmet and mounting of a set of electrodes on the predetermined electrode positions on the helmet;
(e) continuous EEG recording using the set of electrodes carried by the helmet, which is worn by the patient on his head;
(f) mapping of the electrical field, as recorded on the scalp of the patient's head using the set of electrodes;
(g) gray matter segmentation;
(h) solution points distribution
(i) Creation of a volume conductor model of the patient's head (= head model);
(j) calculating the charge distribution present in the brain and responsible for the electrical field, based on the inverse solution of Maxwell's equations;

Note that the approach of Figure 2 allows determination of the spatial location and/or extent of at least one electrical source within the brain from the computed charge distribution, but importantly without performing any tedious 3D optical scanning of the helmet 1. This is because the position of each electrode 2 is well-known from the 3D design data of the helmet 1 and can thus be considered during said computation.

The 3D design data used for fabricating the helmet 1 by 3D printing, including the individual electrode positions, are determined based on existing patient-specific 3D anatomical data measured from a patient's head using MRI, as illustrated in Figure 2. In other words, the position of each electrode 2 is not only well-known but deliberately chosen to perform a certain task, for example accurately determining the location of an unknown source within the head of the patient by ESI or accurately stimulation a particular region of interest (ROI) that has been identified by a clinician in the MRI images.

Currently, stimulation electrodes 2 are often supplied with electric current by means of a current source 28, as schematically illustrated in Figure 3. In such a case, if the superficial conductivity on the scalp 31 increases (which can be modeled by respective variable resistances R_{Sij} between single electrodes 2 Ei, as shown in Figure 3), the electronic current control will regulate the electrical drive voltage downward to keep the electrical current I0 flowing through the stimulation electrode 2 E1 constant (current control). In such a situation, however, less current will reach the brain 30, because a greater part of the injected current is flowing only superficially (towards electrodes E2-E5 via the respective resistive paths modeled by resistors R_{Sij}) but not into the brain 30. This effect, however, will be detrimental for the desired therapeutic effect of the electrical stimulation performed inside the brain 30. This is because, as a result of the smaller current injection into the brain 30, a smaller electric field will be formed inside the brain 30, resulting in less effective stimuli. In extreme cases, with heavy sweating of the patient, it may even be the case that 99% of the injected current flows off superficially and thus less than 1% actually reaches deep layers inside the brain 30.

From another point of view, this situation of electrode stimulation can be imagined as injecting current by means of a current source 28 into an electrical parallel circuit of two resistors R1 and R2, as illustrated in Figure 5: The first electrical resistance R1 corresponds to the almost invariable resistance, which is formed by the current path into the brain 30. The second resistance R2, on the other hand, corresponds to the superficial current paths that exists between the stimulation electrode and potential current sinks (for example, a sensing electrode 2, 34, namely E2/E3/E4/E5, as shown in Figure 3). If a known current I0 is injected and the superficial conductivity on the scalp 31 increases such that R2 decreases, then the current flow I1 through the first resistor R1 will decrease and so will the amount of current injected into the brain 30.

The proposed approach detailed before, which consists in considering such changes of the scalp conductivity, can therefore be understood that by using an electrical measurement on the surface of the patient's skull, the resistance R2 can be determined such that, in terms of control, the current flow I0 delivered by the stimulation electrode E1 (cf. Figure 3) can be adjusted in such a way that the current flow I1 into the brain 30 (cf. Figure 5) can be kept constant and thus a constant electrostimulation can be maintained within the brain 30 during a stimulation session. As detailed before, the helmet 1, more precisely its controller 7 can perform such electrical surface conductivity measurements and correspondingly adapt the electrical stimulation current I0 delivered through one particular stimulation electrode E1, as illustrated in Figure 3.

This approach also makes it possible to provide a kind of "proof"/"sanity check" as to whether the injected current flow I0 actually results in the desired electrostimulation within the brain 30 or not. Consider the worst case scenario: It would be conceivable that over a time span of one hour a high current flow I0 would be delivered by the stimulation electrodes 2 of the electrode helmet 1, but since there is a high conductivity on the scalp 30, this current flow would not efficiently reach deeper areas of the patient's brain 30, which would mean that the therapeutic effect of the electrostimulation would be effectively lost, although a lot of current had been applied.

The adjustment of the stimulation current I0 injected into the scalp 29 can also be carried out dynamically, for example, during a stimulation session, or at the beginning of such a session as part as a series of such stimulation sessions, by the controller 7 of the helmet 1. Thereby, variations of the electrical conductivity of the scalp 29 of the patient can be compensated for.

In addition, the impedance measurement can be designed in such a way that the electrostimulation is interrupted, in case a control (in particular impedance) measurement, performed intermediately with the at least one source electrode 2, 33 E1 and the at least one sensing electrode 2, 34 (E2, E3, E4, E5 in Figure 3), has revealed that the impedance has decreased too much (e.g. below a threshold value) or the conductivity has increased too much (e.g. above a threshold value).

Typically, >90% of the injected current will go through the scalp 29. As the conductivity of the scalp 29 varies a lot from patient to patient, the achieved electrostimulation can be significantly affected, if the variation of the conductivity of the scalp 29 is not compensated for. Therefore, a four-probe-measurement-scheme (sometimes called "four-probe-method") can be used to measure the conductivity of the scalp 29 of the patient using the electrode helmet 1, as illustrated in Figure 4: For this purpose, four of the electrodes 2 of the helmet 1, which are arranged more or less in a straight line, may be employed for such a measurement by placing them at equal spacings s from each other, as shown in Figure 4. The two outer of the four electrodes 2 E1 and E4 then serve to deliver a measurement current Iₘ to the scalp 29 while with the inner two electrodes 2 E2 and E3, a voltage drop ΔV resulting from the measurement current Iₘ can be measured. The slope of the so-obtained ΔV/I-curve provides the impedance of the scalp 29, which is modeled as a thin film, similar to 4-point-probe measurements conducted in the semiconductor industry. In particular, a sheet resistance Rₛ can then be calculated for the scalp 29 using the approximate equation: Rₛ = 4.53236 ΔV/ Iₘ with Rₛ the sheet resistance, ΔV the change in voltage measured between the inner probe electrodes 2 E2 and E3, and Iₘ the mentioned measurement current applied between the outer probe electrodes 2 E1 and E4. The sheet resistance is generally measured using the units Ω/□ (ohms per square), to differentiate it from bulk resistance. The above equation is only valid if the material being tested is no thicker than 40% of the spacing s between the probes and if the lateral size of the sample is sufficiently large. Both factors can be fulfilled by a suitable electrode arrangement on the helmet 1.

Accordingly, the approach presented here can result in a method for a dynamic adaptation of an electrical stimulation current I0 as applied in an electrostimulation in dependence of an electrical impedance measurement (in particular performed as a four-probe-measurement as laid out above), wherein the electrical impedance measurement has been or is carried out with the aid of a source electrode 2, 33 (E1 in Figure 3) and at least one, preferably several, sensing electrodes 2, 34 (E2, E3, E4 and E5 in Figure 3). All of these electrodes 2, 34 may be carried by a helmet 1 as described herein.

In summary, several methods and devices have been proposed for improved electroencephalography source imaging (ESI) and/or improved and tailored electrostimulation. The approach is based on an electrode helmet 1 that carries a set of electrodes 2 at precisely known and predefined spatial positions, in particular because each electrode 2 is held in place by a respective electrode holder 4 of the helmet 1. As the helmet 1, in particular said electrode holders 4, can be 3D-printed based on 3D design data which are based on patient-specific 3D anatomical data, the helmet 1 offers a platform for patient-specific recording of EEG signals and/or electrostimulation. Accordingly, tedious 3D optical scanning of each electrode 2 position is no longer necessary, such that highly accurate ESI and electrostimulation are possible as soon as the patient is wearing the helmet 1 in the intended well-defined orientation on his head (cf. Figure 2).

### List of reference numerals

- 1: electrode helmet (carrying 2)
- 2: electrode (this may be a recording and/or stimulation and/or source and/or sensing electrode, in particular in the form of a brush electrode)
- 3: (spatial) position (in 3D of 2 within a coordinate system of 1).
- 4: electrode holder (holds 2 in place, relative to 1 / to the skull of the patient; this holder may press 2 into the scalp of the patient with a defined contact force)
- 5: inner shell (of 1)
- 6: inner contour surface (of 1/5)
- 7: controller
- 8: electronic unit (for EEG recording and/or for electrostimulation)
- 9: brush electrode
- 10: outer shell (of 1)
- 11: contact pin (for electrically contacting 2)
- 12: electrode connector
- 13: cerebrospinal fluid (CSF = liquor cerebrospinalis)
- 14: electrical wiring
- 15: electrical stimulation and/or recording device
- 16: recess (for the patient's ear)
- 17: (lower) rim (of 1)
- 18: cable
- 19: wireless data connection
- 20: cable connector (of 1, for connection to 8)
- 21: wireless communication interface (e.g., bluetooth)
- 22: brush electrode
- 23: feed channel (for conductive gel)
- 24: spring / bending beam
- 25: contact force
- 26: recess / opening (for weakening 24)
- 27: slit (in 10 for defining 24)
- 28: current source (for injecting a current into via 2 into 30)
- 29: skull
- 30: brain
- 31: scalp
- 32: region of interest = ROI (e.g. possible location of source or region within 30 to be electrostimulated)
- 33: source electrode
- 34: sensing electrode

## Claims

1. **Method for high-accuracy electroencephalography source imaging (ESI),** wherein a spatial location and/or extent of at least one electrical source is computed from a recorded electroencephalography signal, *preferably and outputted as a digital source image,* **characterized in that,**
- according to an optimization goal and/or according to a medical objective, a respective electrode position (3) for each electrode (2) of a set of recording electrodes (2) is fixated on an electrode helmet (1), the helmet (1) therefore being usable for recording the electroencephalography signal and the helmet (1) carrying said electrodes (2), and
- the so predefined electrode positions (3) are considered during the computation of the spatial location and/or extent of the at least one electrical source,
- in particular without performing any 3D-measurement of the electrode positions (3).

2. Method according to claim 1,
- wherein the electrode positions (3) are determined based on existing patient-specific 3D anatomical data measured from a patient's head, in particular anatomical data describing the spatial distribution of the patient's brain, skull and scalp,
- preferably wherein the 3D anatomical data have been measured using a medical imaging technique,
- most preferably using magnetic resonance imaging (MRI).

3. Method according to claim 1 or claim 2,
- wherein the computation uses a patient-specific head model which is derived from, in particular the, 3D anatomical data measured from a head of a patient,
- preferably wherein the brain of said patient is imaged with the method to find at least one electroencephalography source.

4. **Method for preparing an electrode helmet (1)**
- for patient-specific electrical stimulation of a particular region of interest (ROI) inside the brain of a patient and/or
- for recording of nerve signals emanating from inside the brain of a patient,
each time using a respective set of electrodes (2) carried by said electrode helmet (1),
**the method comprising,**
- step A): deriving 3D design data for the helmet (1), including respective electrode positions (3) for each electrode (2) of the set, from existing patient-specific 3D anatomical data measured from the patient's head,
- in particular based on an optimization goal and/or according to a medical objective, and
- step B): arranging and/or mounting a number of m electrodes (2) on the helmet (1) in a patient-specific and/or customized arrangement that is defined by or derived from the 3D design data obtained in step A).

5. Method according to one of the claims 2 to 4,
- wherein the 3D anatomical data have been or are measured using a medical imaging technique,
- most preferably using magnetic resonance imaging (MRI),
- even more preferably using diffusion-weighted magnetic resonance imaging (DWI).

6. Method according to any one of claims 2 to 5,
- wherein the anatomical data comprise image data measured with at least two different medical imaging techniques,
- most preferably a first set of data measured with diffusion-weighted magnetic resonance imaging (DW-MRI) and a second set of data measured with structural magnetic resonance imaging.

7. Method according to any one of the claims 2 to 6,
- wherein, in particular in step A), the respective electrode positions (3) for each electrode (2) of the set are calculated based on a patient-specific head model which is derived from said 3D anatomical data,
- preferably wherein said patient-specific head model comprises multiple spatial distributions of different layers, wherein a respective electrical conductivity (e.g. standard / literature) is assigned to each layer.

8. Method according one of the claims 2 to 7,
- wherein, in particular in step A), the respective electrode positions (3) for each electrode (2) of the set are calculated based on an optimization goal, in particular namely
- maximizing the effectiveness of an electrical stimulation of at least one pre-defined region of interest inside the patient's brain
and/or
- maximizing the resolution when recording EEG signals from a particular source/region of interest within the patient's brain.

9. Method according to any one of the claims 4 to 8,
- wherein step B) comprises 3D-printing of at least parts of said helmet (1) for fixing/predefining each of said electrode positions (3) with respect to the brain of the patient for whom the helmet (1) is custom-made.

10. Method according to any one of the claims 4 to 9,
- wherein step B) comprises defining, in particular by 3D-printing or by adapting parts of said helmet (1), an inner contour surface (6) of said helmet (1) based on a specific shape and size of the skull of the patient,
- in particular such that the helmet (1) can only be worn by the patient on her/his head in an unchangeable orientation and/or position relative to the skull of the patient and/or such that the helmet (1) shows a patient-specific design.

11. Method according to any one of the claims 4 to 10,
- wherein the electrodes (2) are mounted in electrode holders (4), in particular which are provided separately or as part of a shell (5, 10) of said helmet (1), and
- wherein the shape and/or position and/or orientation of each of the electrode holders (4) is defined by said 3D design data.

12. **Method for preparing a reliable and desired electrostimulation of a brain of a patient,**
- in particular according to one of the preceding claims 4 to 11,
- wherein prior to or simultaneous to performing electrostimulation with a set of stimulation electrodes (2), preferably carried by an/the electrode helmet (1), an electrical surface conductivity measurement, preferably an electrical impedance measurement, is carried out using at least one source electrode (2, 33) and at least one electrical sensing electrode (2, 34), preferably carried by said electrode helmet (1), wherein
- during the measurement, the at least one source electrode (2, 33) injects a known electrical measurement current into the scalp of the patient and
- a voltage or current is recorded by the at least one electrical sensing electrode (2, 34).

13. Method according to claim 12,
- wherein the measured electrical surface conductivity is considered in an electrostimulation performed with the electrode helmet (1) afterwards, in particular by adapting / correcting at least one stimulation current delivered by the electrode helmet (1) based on the electrical surface conductivity measurement and/or
- wherein a plurality of sensing electrodes (2, 34), which are employed during said measurement, are arranged in a precisely predetermined geometrical arrangement relative to one another on the electrode helmet (1) and thus relative to the skull and brain of the patient and
- a spatial distribution of a surface conductivity of the scalp of the patient is calculated from the measurement by considering said geometrical arrangement.

14. **Electrode helmet (1)** with patient-specific design,
- in particular prepared for patient-specific electrical stimulation with a method according to one of the claims 4 to 11 and/or wherein
- the helmet (1) carries a set of stimulation electrodes (2) designed and configured for producing an electrostimulation inside the brain of a patient wearing said helmet (1) on his head,
- wherein the helmet (1) features
- at least one source electrode (2, 33) and at least one electrical sensing electrode (2, 34) and
- an electronic controller (7), wherein
- the controller (7) is configured to perform an electrical surface conductivity measurement, preferably an electrical impedance measurement and/or based on a four-probe-measurement-scheme, using the at least one source electrode (2, 33) and the at least one electrical sensing electrode (2, 34).

15. Electrode helmet (1) according to claim 14,
- wherein the controller (7) is configured to implement a method according to one of the claims 12 or 13,
- most preferably, wherein the controller (7) is further configured to, repeatedly and/or dynamically, adjust at least one electrical stimulation current, as delivered by one of said stimulation electrodes (2),
- in particular during an electrostimulation performed with the helmet (1) by considering a momentary result of said measurement.
